Europäisches Patentamt

**European Patent Office**

Office européen des brevets

(19)

(11) Veröffentlichungsnummer : **0 039 806**
**B1**

## (12) EUROPÄISCHE PATENTSCHRIFT

(45) Veröffentlichungstag der Patentschrift :
**16.11.83**

(51) Int. Cl.³ : **A 61 K 7/13**

(21) Anmeldenummer : **81103091.5**

(22) Anmeldetag : **24.04.81**

(54) **Haarfärbemittel.**

(30) Priorität : **02.05.80 DE 3016904**

(43) Veröffentlichungstag der Anmeldung :
**18.11.81 Patentblatt 81/46**

(45) Bekanntmachung des Hinweises auf die Patenterteilung : **16.11.83 Patentblatt 83/46**

(84) Benannte Vertragsstaaten :
**AT BE CH DE FR GB IT LI LU NL SE**

(56) Entgegenhaltungen :
**CH A 479 302**
**US A 1 932 901**
**US A 2 552 354**
**US A 3 970 423**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Rose, David, Dr.**
**Holbeinweg 7**
**D-4010 Hilden (DE)**
Erfinder : **Maak, Norbert, Dr.**
**Liebigstrasse 18**
**D-4040 Neuss (DE)**

## Haarfärbemittel

Gegenstand der Erfindung sind Mittel zur oxidativen Färbung von Haaren auf Basis eines N-Hydroxyalkyl-p-phenylendiamins als Entwicklerkomponente.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen, wie p-Phenylendiaminderivate, Diaminopyridine, 4-Amino-pyrazolon-derivate, heterocyclische Hydrazone eingesetzt. Als sogenannte Kupplerkomponenten werden m-Phenylendiaminderivate, Phenole, Naphthole, Resorcinderivate und Pyrazolone genannt.

Gute Oxidationshaarfarbstoffkomponenten müssen in erster Linie folgende Voraussetzungen erfüllen :

Sie müssen bei der oxidativen Kupplung mit den jeweiligen Entwickler- bzw. Kupplerkomponenten die gewünschten Farbnuancen in ausreichender Intensität ausbilden. Sie müssen ferner ein ausreichendes bis sehr gutes Aufziehvermögen auf menschlichem Haar besitzen und sie sollen darüber hinaus in toxikologischer und dermatologischer Hinsicht unbedenklich sein.

Die üblicherweise als Entwicklersubstanzen verwendete Verbindungsklasse der substituierten bzw. unsubstituierten p-Phenylendiamine besitzt den Nachteil, daß sie bei einer Reihe von Personen Sensibilisierungen und in deren Gefolge schwere Allergien hervorruft. Die zur Vermeidung dieser dermatologischen Nachteile in neuerer Zeit vorgeschlagenen Entwicklersubstanzen können in ihren anwendungstechnischen Eigenschaften nicht immer voll befriedigen.

Es bestand daher bei der Suche nach brauchbaren Oxidations haarfarbstoffen die Aufgabe, geeignete Komponenten aufzufinden, die frei von den geschilderten Nachteilen sind und die genannten Voraussetzungen in optimaler Weise erfüllen.

Es wurde gefunden, daß Haarfärbemittel auf Basis von Oxidationsfarbstoffen mit einem Gehalt an N-(2-Hydroxy-propyl)-p-phenylendiamin, sowie dessen anorganischen oder organischen Salzen als Entwicklersubstanzen und den in Oxidationshaarfarben üblichen Kupplersubstanzen den gestellten Anforderungen in besonders hohem Maße gerecht werden. « N-(2-Hydroxylpropyl)-p-phenylendiamin ist aus US-A-2.552.354 als Vorstufe für die Herstellung des Diazoniumsalzes zur Erzeugung von Azofarbstoffen bekannt. Eine Verwendung als Vorstufe für Oxidationsfarbstoffe wird in dieser Druckschrift nicht erwähnt. »

N-(2-Hydroxypropyl)-p-phenylendiamin kann nach den üblichen Methoden der organischen Synthese erhalten werden. Eine geeignete Herstellungsweise ist im Beispielteil der Beschreibung wiedergegeben.

Beim Einsatz als Entwicklerkomponente liefert das N-(2-Hydroxypropyl)-p-phenylendiamin mit den im allgemeinen für die Oxidationshaarfärbung verwendeten Kupplersubstanzen ein breites Spektrum sehr intensiver Farbnuancen, wie sie mit diesen Kupplern und den bisher bekannten Entwicklern nicht erzielbar waren. Demzufolge stellen Haarfärbemittel mit einem Gehalt an N-(2-Hydroxypropyl)-p-phenylendiamin eine wesentliche Bereicherung der Möglichkeiten zur oxidativen Haarfärbung dar. Darüber hinaus zeichnen sich die mit dieser Substanz erzielten Färbungen durch überlegene Echtheitseigenschaften aus. Weiterhin sind N-(2-Hydroxy-propyl)-p-phenylendiamin und seine Salze toxikologisch und dermatologisch unbedenklich und besitzen eine gute Löslichkeit in Wasser sowie eine gute Lagerstabilität.

In den erfindungsgemäßen Haarfärbemitteln kann N-(2-Hydroxypropyl)-p-phenylendiamin entweder als solches oder in Form seiner Salze mit anorganischen oder organischen Säuren eingesetzt werden, zum Beispiel als Chlorid, Sulfat, Phosphat, Acetat, Propionat, Lactat oder Citrat.

Als weitere Farbstoffkomponenten sind in den erfindungsgemäßen Haarfärbemitteln die üblicherweise als Kupplersubstanzen verwendeten Verbindungen enthalten. Beispiele für derartige Kupplerkomponenten sind : α-Naphthol, o-Kresol, m-Kresol, 1,6-Dimethylphenol, 2,5-Dimethylphenol, Brenzcatechin, Pyrogallol, 1,5- bzw. 1,7-Dihydroxynaphthalin, 5-Amino-2-methylphenol, m-Aminophenol, 2,4-Diaminoanisol, m-Toluylendiamin, Resorcin, Resorcinmonomethylether, m-Phenylendiamin, 1-Phenyl-3-methyl-pyrazolon-5, 1-Amino-3-acetylacetamino-4-nitrobenzol oder 1-Amino-3-cyanacetylamino-4-nitrobenzol, 2-Methylresorcin, 2,4-Diaminophenol, 3,5-Diamino-2-methoxybenzol, 1-Ethoxy-2-bis-(β-hydroxy-ethyl)-imino-4-aminobenzol, 2,4-Dichlor-3-aminophenol, 1-Phenyl-3-ethylureidopyrazolon-5, 1,3-Bis-(2,4-diaminophenoxy)-propan, 4-Chlorresorcin.

In den erfindungsgemäßen Haarfärbemitteln werden die Entwicklerkomponenten im allgemeinen in etwa molaren Mengen, bezogen auf die verwendeten Kupplersubstanzen, eingesetzt. Wenn sich auch der molare Einsatz als zweckmäßig erweist, so ist es jedoch nicht nachteilig, wenn die Entwicklerkomponente in einem gewissen Überschuß oder Unterschuß zum Einsatz gelangt.

Es ist nicht erforderlich, daß die Entwicklerkomponente und die Kupplersubstanz einheitliche Produkte darstellen, vielmehr können sowohl die Entwicklerkomponente als auch die Kupplersubstanz Gemische der vorstehend genannten Verbindungen darstellen.

Darüber hinaus können die erfindungsgemäßen Haarfärbemittel andere bekannte und übliche Entwicklerkomponenten, sowie auch gegebenenfalls übliche direktziehende Farbstoffe im Gemisch enthalten, falls dies zur Erzielung gewisser Farbnuancen erforderlich ist.

Die oxidative Kupplung, das heißt die Entwicklung der Färbung, kann grundsätzlich wie bei anderen Oxidationshaarfarbstoffen auch, durch Luftsauerstoff erfolgen. Zweckmäßigerweise werden jedoch chemische Oxidationsmittel eingesetzt. Als solche kommen insbesondere Wasserstoffperoxid oder dessen Anlagerungsprodukte an Harnstoff, Melamin und Natriumborat, sowie Gemische aus derartigen Wasserstoffperoxidanlagerungsverbindungen mit Kaliumperoxiddisulfat in Betracht.

Die erfindungsgemäßen Haarfärbemittel werden für den Einsatz in entsprechende kosmetische Zubereitungen, wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwicklerkombination beträgt 0,2 bis 5 Gewichtsprozent, vorzugsweise 1-3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind zum Beispiel Netz- oder Emulgiermittel vom anionischen oder nicht-ionogenen Typ, wie Alkylbenzolsulfonate, Fettalkoholsulfate, Alkylsulfonate, Fettsäurealkanolamide, Anlagerungsprodukte von Ethylenoxid an Fettalkohole, Verdickungsmittel, wie Methylcellulose, Stärke, höhere Fettalkohole, Paraffinöl, Fettsäuren, ferner Parfümöle und Haarpflegemittel, wie Pantothensäure und Cholesterin zu nennen.

Die genannten Zusatzstoffe werden dabei in den für diese Zwecke üblichen Mengen eingesetzt, wie zum Beispiel Netz- und Emulgiermittel in Konzentrationen von 0,5-30 Gewichtsprozent und Verdickungsmittel in Konzentrationen von 0,1-25 Gewichtsprozent, jeweils bezogen auf die gesamte Zubereitung.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8-10 erfolgen. Die Anwendungstemperaturen bewegen sich dabei im Bereich von 15 bis 40 °C. Nach einer Einwirkungsdauer von circa 30 Minuten wird das Haarfärbemittel vom zu färbenden Haar durch Spülen entfernt. Hernach wird das Haar mit einem milden Shampoo nachgewaschen und getrocknet.

Die mit den erfindungsgemäßen Haarfärbemitteln erzielbaren Farbtöne zeigen unter Einsatz unterschiedlicher Entwickler- und Kupplerkomponenten eine außerordentliche Variationsmöglichkeit, die von dunkelbraun bis rubinrot und violett reicht. Die erzielten Färbungen haben gute Licht-, Wasch- und Reibechtheitseigenschaften und lassen sich leicht mit Reduktionsmitteln wieder abziehen.

Die Verwendung von N-hydroxyalkylsubstituierten p-Phenylendiaminen als Komponenten in Oxidationshaarfarben ist aus der US-PS 1 932 001 und aus der DE-AS 22 40 495 bekannt. N-(2-Hydroxypropyl)-p-phenylendiamin wird jedoch in keiner der beiden Druckschriften als chemisches Individuum erwähnt. Dem entsprechend findet sich dort auch kein Hinweis auf die überraschenden und herausragenden Lichtechtheitseigenschaften der Färbungen, die mit N-(2-Hydroxy-propyl)-p-phenylendiamin als Entwicklerkomponente in Oxidationsfarben erzielt werden können.

Die nachfolgenden Beispiele sollen den Erfindungsgegenstand näher erläutern, ohne ihn jedoch hierauf zu beschränken.

## Beispiele

Das in den nachfolgenden Beispielen für erfindungsgemäße Haarfärbemittel einzusetzende N-(2-Hydroxypropyl)-p-phenylendiamin wurde als Dihydrochlorid nach den üblichen Methoden der organischen Synthese hergestellt. Die gewählte Herstellung wird nachstehend kurz beschrieben :

A) N-(2-Hydroxypropylamino)-4-nitrobenzol

Ein Gemisch aus 21,2 g (0,15 Mol) 4-Fluornitrobenzol, 15 g (0,2 Mol) 1-Amino-2-propanol und 10,4 g Kaliumcarbonat wurde in 25 ml Dimethylformamid 2 Stunden lang zum Rückflußkochen erhitzt. Nach dem Abkühlen wurde das Reaktionsgemisch in Eiswasser gegossen und der Niederschlag abfiltriert. Durch Umkristallisieren aus Toluol wurden gelbe Kristalle mit Schmp. 79 °C erhalten.

B) N-(2-Hydroxypropyl)-p-phenylendiamin-dihydrochlorid

15 g N-(2-Hydroxypropylamino)-4-nitrobenzol wurden in 100 ml Ethanol in Gegenwart von 5 Gewichtsprozent Palladium auf Kohle bei 25 °C und einem Wasserstoffdruck von 2 bar hydriert. Nach dem Ende der Wasserstoffaufnahme wurde der Katalysator abfiltriert, das Filtrat mit verdünnter Salzsäure angesäuert und zur Trockne eingeengt. Graue Kristalle, Schmp. 230 °C.

In den folgenden Beispielen wurde N-(2-Hydroxypropyl)-p-phenylendiamin-hydrochlorid in Kombination mit den nachstehenden Verbindungen als Kupplersubstanzen eingesetzt :

K 1 : α-Naphthol
K 2 : 1,7-Dihydroxynaphthalin
K 3 : Resorcin
K 4 : 2-Methylresorcin
K 5 : m-Aminophenol

3

K 6 : 5-Amino-2-methylphenol
K 7 : 2,4-Dichlor-3-aminophenol
K 8 : 1-Phenyl-3-methylureido-pyrazolon.

Die erfindungsgemäßen Haarfärbemittel wurden in Form einer Cremeemulsion eingesetzt. Dabei wurden in eine Emulsion aus

10 Gewichtsteilen Fettalkoholen der Kettenlänge $C_{12}$-$C_{18}$,
10 Gewichtsteilen Fettalkoholsulfat (Natriumsalz) Kettenlänge $C_{12}$-$C_{18}$ und
75 Gewichtsteilen Wasser

jeweils 0,01 Mol N-(2-Hydroxypropyl)-p-phenylendiamin-dihydrochlorid als Entwicklersubstanz und 0,01 Mol der in der nachstehenden Tabelle aufgeführten Kupplersubstanzen eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde mit 1 %iger Wasserstoffperoxidlösung als Oxidationsmittel durchgeführt, wobei zu 100 Gewichtsteilen der Emulsion 10 Gewichtsteile Wasserstoffperoxidlösung gegeben wurden. Die jeweilige Färbecreme mit Zusatz von Oxidationsmitteln wurde auf zu 90 % ergrautes, nicht besonders vorbehandeltes Menschenhaar aufgetragen und dort 30 Minuten belassen. Nach Beendigung des Färbeprozesses wurde das Haar mit einem üblichen Haarwaschmittel ausgewaschen und anschließend getrocknet. Die dabei erhaltenen Färbungen sind nachstehender Tabelle 1 zu entnehmen.

Tabelle 1

| Beispiel | Kuppler | Mit 1 %iger $H_2O_2$-Lösung erhaltener Farbton |
|---|---|---|
| 1 | K 1 | schwarzblau |
| 2 | K 2 | schwarzblau |
| 3 | K 3 | braunschwarz |
| 4 | K 4 | braungrau |
| 5 | K 5 | schwarzviolett |
| 6 | K 6 | dunkelviolett |
| 7 | K 7 | violettschwarz |
| 8 | K 8 | dunkelrubin |

Vergleichsversuche zur Lichtechtheit

Die Lichtechtheitseigenschaften wurden nach DIN 54 004 geprüft. Zur Herstellung der Färbungen wurden Färbemittel mit folgenden Kupplersubstanzen hergestellt :

K 1 : α-Naphthol
K 3 : Resorcin
K 6 : 5-Amino-2-methylphenol
K 9 : 1-Phenyl-3-acetamidopyrazolon.

Als Entwicklerkomponenten wurden folgende p-Phenylendiamine verwendet :

E 1 : N-(2-Hydroxypropyl)-p-phenylendiamin
E 2 : N-(2-Hydroxy-2-methylpropyl)-p-phenylendiamin
E 3 : N-(6-Hydroxyhexyl)-p-phenylendiamin,

wobei E 2 und E 3 Vergleichssubstanzen darstellen. Die bei der Prüfung nach DIN 54 004 erhaltenen Ergebnisse sind in der nachstehenden Tabelle 2 wiedergegeben.

Tabelle 2

|  | E 1 | E 2 | E 3 |
|---|---|---|---|
| K 1 | 4 | 2-3 | 2 |
| K 3 | 4-5 | 3 | 2 |
| K 6 | 4 | 2 | 2 |
| K 9 | 3-4 | 2 | 2 |

Die Tabelle 2 läßt deutlich die überragenden Lichtechtheitseigenschaften der mit n-(2-Hydroxypropyl)-p-phenylendiamin als Entwicklerkomponente erzielten Färbungen erkennen.

**Ansprüche**

1. Haarfärbemittel auf Basis von Oxidationsfarbstoffen, gekennzeichnet durch einen Gehalt an N-(2-Hydroxypropyl)-p-phenylendiamin sowie dessen anorganischen oder organischen Salzen als Entwicklersubstanz und den in Oxidationshaarfarben üblichen Kupplersubstanzen.

2. Haarfärbemittel nach Anspruch 1, gekennzeichnet durch einen Gehalt an Entwickler-Kuppler-Kombination von 0,2 bis 5 Gewichtsprozent, vorzugsweise 1 bis 3 Gewichtsprozent.

**Claims**

1. Hair dyes based on oxidation dyes, characterized by a content of N-(2-hydroxypropyl)-p-phenylene diamine and its inorganic or organic salts as developer substance and the coupler substances normally used in oxidation hair dyes.

2. Hair dyes as claimed in Claim 1, characterized by a content of from 0.2 to 5 % by weight and preferably from 1 to 3 % by weight of developer-coupler combination.

**Revendications**

1. Agent de teinture pour cheveux à base de colorants d'oxydation, caractérisé par une teneur en N-(2-hydroxypropyl)-p-phénylène diamine ainsi qu'en ses sels minéraux ou organiques comme substance de développement et en les substances de couplage usuelles dans les teintures oxydantes pour cheveux.

2. Agent de teinture pour cheveux selon la revendication 1, caractérisé par une teneur en la combinaison agent de développement-coupleur de 0,2 à 5 % en poids, de préférence de 1 à 3 % en poids.